Europäisches Patentamt

European Patent Office

Office européen des brevets

(11) Publication number: **0 323 900 B1**

(12) **EUROPEAN PATENT SPECIFICATION**

(45) Date of publication of patent specification: **11.08.93**

(51) Int. Cl.5: **C12N 7/04**, C12N 15/00, A61K 39/125, A61K 39/13, A61K 39/29

(21) Application number: **89300060.4**

(22) Date of filing: **05.01.89**

(54) **Attenuated viruses.**

(30) Priority: **05.01.88 GB 8800100**

(43) Date of publication of application:
**12.07.89 Bulletin 89/28**

(45) Publication of the grant of the patent:
**11.08.93 Bulletin 93/32**

(84) Designated Contracting States:
**AT BE CH DE ES FR GR IT LI LU NL SE**

(56) References cited:

Nucleic Acids Research, vol. 12, no. 20, 1984, pp. 7787-7792, A.J. Cann et al.

Nucleic Acids Research, vol. 11, no. 16, 1983, pp. 5629-5643, G. Stanway et al.

J. of General Virology, vol. 69, 1988, pp. 1091-1096, GB, P.D. Minor et al.

UCLA Symposium Molecular Cellular Biology, (New series), vol. 54, 1987, Eds. M.A. Brinton, pp. 437-452, A. Nomoto et al.

(73) Proprietor: **ALMOND, Jeffrey William Department of Microbiology University of Reading London Road Reading RG1 5AO(GB)**

Proprietor: **Skinner, Michael Anthony Medical Research Council Laboratory of Molecular Biology Hills Road Cambridge CB2 2OH(GB)**

Proprietor: **Racaniello, Vincent Department of Microbiology College of Physicians & Surgeons of Columbia University 701 West 168th Street New York, NY 10032(US)**

Proprietor: **Minor, Philip David National Institute for Biological Standards & Control Blanche Lane South Mimms Potters Bar Herts EN6 3OG(GB)**

Rank Xerox (UK) Business Services
(3.10/3.6/3.3.1)

EP 0 323 900 B1

(72) Inventor: **ALMOND, Jeffrey William**
**Department of Microbiology University of**
**Reading London Road**
**Reading RG1 5AO(GB)**
Inventor: **Skinner, Michael Anthony**
**Medical Research Council Laboratory of Mo-**
**lecular Biology Hills Road**
**Cambridge CB2 2OH(GB)**
Inventor: **Racaniello, Vincent**
**Department of Microbiology College of Phy-**
**sicians & Surgeons of Columbia University**
**701 West 168th Street**
**New York, NY 10032(US)**
Inventor: **Minor, Philip David**
**National Institute for Biological Standards &**
**Control Blanche Lane South Mimms**
**Potters Bar Herts EN6 3OG(GB)**


(74) Representative: **Woods, Geoffrey Corlett et al**
**J.A. KEMP & CO. 14 South Square Gray's Inn**
**London WC1R 5EU (GB)**

**Description**

This invention relates to the construction of vaccines against rhinoviruses and enteroviruses, particularly polioviruses, by the introduction of defined mutations into their genomes. These mutations attenuate the virulence of wild type viruses and can further attenuate existing live attenuated vaccine virus strains, thereby making them less likely to revert to virulence.

At the present time, the only vaccines routinely used against enterovirus and rhinovirus infections are those against poliomyelitis. Of these the live attenuated vaccines developed by Sabin in the 1950's have found greatest use throughout the world. Vaccine strains derived from each of the three poliovirus serotypes (P1, P2 and P3) were prepared by passage of wild type viruses in cell cultures and whole animals until attenuated strains were obtained. These attenuated viruses are substantially less able to cause poliomyelitis in humans than the original wild type strains. They are administered orally and replicate in the gut to induce a protective immune response.

Although these vaccines are generally regarded as safe, their use is associated with a low level of paralysis in vaccinees. This is most often associated with type 2 and type 3 serotypes and rarely, if ever, with type 1. There is therefore a requirement for improved type 2 and type 3 vaccines which would be comparable in safety to the excellent type 1 strain. There is also a requirement for vaccines against other enteroviruses, e.g. echo, coxsackie and hepatitis A, and against rhinoviruses.

The Sabin vaccine strains were developed by essentially empirical procedures. The genetic basis of their attenuation is not properly understood. Over the past few years, however, scientists have employed a number of molecular biological techniques in an attempt to elucidate the mechanism by which the neurovirulence of these vaccine strains is reduced. Most of the work has concentrated on serotypes 1 and 3. For both of these the complete nucleotide sequences of the vaccine strains have been compared with those of their neurovirulent progenitors.

In the case of poliovirus type 1, the vaccine strain differs from its progenitor at 47 positions in the 7441 base genome (Nomoto et al, 1982, Proc Natl Acad Sci USA 79 : 5793-5797). All of these are simple point mutations and 21 of them give rise to amino acid changes in virus coded proteins. Although several mutations are thought to contribute to the attenuation phenotype of the vaccine strain, direct evidence has been presented that the mutation of A to G at position 480 in the 5′ non-coding region of the genome has a marked attenuating effect on the virus (Nomoto et al, 1987, UCLA Symp Mol Cell Biol, New Series, Vol 54 (Eds M A Brinton and R R Rueckert), 437-452, New York : Alan R Liss Inc).

Analogous studies on poliovirus type 3 reveal just 10 nucleotide sequence differences in the 7432 base genome between the vaccine and its progenitor strain (Stanway et al, 1984, Proc Natl Acad Sci USA 81 : 1539-1543). Just three of these give rise to amino acid substitutions in virus encoded proteins. The construction of defined recombinants between the vaccine and its progenitor strain has allowed the identification of the mutations which contribute to the attenuation phenotype. One of these is at position 2034 and causes a serine to phenylalanine change in virus protein VP3.

The other mutation of interest is C to U at position 472 in the 5′ non-coding region of the genome. This latter mutation has been observed to revert to the wild type C rapidly upon replication of the virus in the human gut (Evans et al, 1985, Nature 324 : 548-550). This reversion is associated with an increase in neurovirulence. C at position 472 has also been shown to be essential for growth of a mouse/human polio recombinant virus in the mouse brain (La Monica et al, 1986, J Virol 57 : 515-525). Recently, we have observed that at 481 in poliovirus type 2 A changes to G in an analogous fashion upon replication of the type 2 vaccine in the gut of vaccinees.

We have investigated mutations of the wild-type poliovirus type 3 Leon strain at several sites in the 5′ non-coding region approximately spanning nucleotides 450 to 510. We found that poliovirus with a mutation at position 479 or 482 is attenuated but with a mutation at position 480 is non-attenuating. Multiple mutations were frequently lethal. However, poliovirus with a double mutation at positions 472 and 482 was more highly attenuated than poliovirus with a single mutation at either position.

The findings can be extrapolated to all polioviruses. Indeed, they may be extrapolated to other enteroviruses and rhinoviruses. Mutations at sites of other enteroviruses and rhinoviruses corresponding to position 479 and/or 482 and, optionally, position 472 of poliovirus type 3 Leon strain can lead to attenuation. There is a relatively high degree of homology between the genome RNA of all enteroviruses and rhinoviruses. The positions of another strain of enterovirus or rhinovirus corresponding to positions 472, 479 and 482 of poliovirus type 3 Leon strain (based on the numbering used in the Stanway et al paper already referred to) can be determined by lining up the sequences of the genomic RNA of the strains.

Accordingly the invention relates to attenuated enteroviruses and rhinoviruses having an attenuating mutation at least at a position which is, or corresponds with, postion 479 and/or 482, and optionally also position 472, of the genome of poliovirus type 3 Leon strain.

The present invention is particularly applicable to polioviruses. We have found in particular that the mutation A to C at position 479 of the genome of poliovirus type 3 Leon strain causes attenuation, as does mutation G to A at position 482. Either or both may be combined with the mutation C to U at position 472. We have further found that poliovirus type 3 with mutations at both positions 472 and 482 is more highly attenuated than viruses with mutations at either one of the positions.

An attenuated poliovirus may be a type 1, type 2 or type 3 poliovirus. Types 2 and 3 are preferred. For types 1 and 2, positions 476, 479 and 469 correspond to positions 479, 482 and 472 respectively of poliovirus type 3. An attenuated type 1 or type 2 poliovirus therefore includes an attenuating mutation at position 476 and/or 479 and, optionally, also at position 469. These mutations may be as above for type 3.

An attenuated virus according to the invention is prepared by a process comprising:

(i) introducing the or each desired mutation by site-directed mutagenesis into a sub-cloned region, which includes the or each position it is wished to mutate, of a cDNA copy of the genome of an enterovirus or rhinovirus;

(ii) reintroducing the thus modified region into the complete cDNA from which the region was derived; and

(iii) obtaining live virus from the cDNA thus obtained.

A mutation can be introduced into a strain of an enterovirus or rhinovirus, for example wild-type virus, by site-directed mutagenesis of a cDNA copy of its genomic RNA. This may be achieved beginning with sub-cloning the appropriate region from an infectious DNA copy of the genome of any of the virus strain, for example a vaccine strain or its progenitor, into the single strand DNA of a bacteriophage such as M13. The virus strain may be a neurovirulent strain but is preferably a vaccine strain. For poliovirus it may be a Sabin, type 3 Leon or type 1 Mahoney strain. The or each desired mutation is then introduced into this sub-cloned cDNA using the technique of oligonucleotide directed mutagenesis.

After the introduction of mutations, the modified sub-cloned cDNAS are reintroduced into the complete cDNA from which they were derived and, for virulence testing in mice, into a cDNA derived from a murine poliovirus derivative known to cause a poliomyelitis type disease in mice (La Monica et al). Live virus is recovered from the mutated full length cDNA by production of a positive sense RNA typically using a T7 promoter to direct transcription in vitro (Van der Werf et al, 1986, Proc Natl Acad Sci, USA 83 : 2330-2334).

The recovered RNA may be applied to tissue cultures using standard techniques (Koch, 1973, Curr Top Microbiol Immunol 61: 89-138). After 4-6 days incubation virus can be recovered from the supernatant of the tissue culture. The level of neurovirulence of the modified virus may then be compared with that of the unmodified virus using a standard LD50 test in mice (La Monica et al) or the WHO approved vaccine safety test in monkeys (WHO Tech Rep Ser 687 : 107-175, 1983).

The attenuated viruses can be used as vaccines. They may therefore be formulated as pharmaceutical compositions further comprising a pharmaceutically acceptable carrier or diluent. Any carrier or diluent conventionally used in vaccine preparations may be employed. For example, the presently used live attenuated poliovirus strains are stabilised in a solution of 1 molar $MgCl_2$ and administered as a mixture of the three serotypes.

The attenuated viruses can therefore be used to prevent an infection attributable to an enterovirus or rhinovirus in a human patient. For this purpose, they may be administered orally, as a nasal spray, or parenterally, for example by subcutaneous or intramuscular injection. A dose corresponding to the amount administered for a conventional live virus vaccine, such as up to $10^6$ $TCID_{50}$ for a Sabin vaccine strain in the case of poliovirus, may be administered.

The following Example illustrates the invention.

EXAMPLE

A HindIII-SstI fragment from a cDNA clone of P3/Leon/37, which includes the first 751 base pairs of the genome, was sub-cloned into the phage vector M13mp9. P3/Leon/37 is the neurovirulent progenitor of the type 3 vaccine strain. Mutations were then introduced into this sub-cloned cDNA fragment using the technique of oligonucleotide directed mutagenesis. The chemically synthesised DNA oligonucleotides used are shown in Table 1 below:

## TABLE 1

| Oligo-nucleotide No | Sequence (5' -3') |
|---|---|
| 1 | GCC-TGC-TCC-ATG-GTT-ATA-TTT-AGC-CGC-ATT |
| 2 | GCC-TGC-TCC-ATG-GTT-ATG-TTT-AGC-CGC-ATT |
| 3 | GCA-GCT-GCC-TGC-CTC-ATT-TTT-AGA-GTT-AGC-CGC-ATT-CAG-C |
| 7 | GCT-GCC-TGC-TTC-ATG-GTT |
| 8 | GCT-GCC-TGC-TTC-ATG-CTT-AGA-ATT-AGC-CGC-A |
| 9 | GCT-GCC-TGC-TGC-ATG-GTT-AGC-ATT-AGC-C |
| 11 | GCT-GCC-TGC-TCC-CAT-GGT-TAG-GAA-TTA-GCC |
| 12 | GCT-GCC-TGC-TAT-TAG-CCG |
| 13 | CCT-GCT-CCA-GGG-TTA-GG |
| 14 | CCT-GCT-CCG-TGG-TTA-GG |

The sequence from base 470 to 484 of the genomic RNA of poliovirus type 3 Leon strain and of mutants derived from this strain is shown in Table 2 below. Mutations are shown by lower case letters. The viability of the strains is also shown, "+" meaning viable and "-" meaning not. Of the viable strains, the parental Leon strain and mutant 14 are virulent. Mutants 7, 8 and 13 are attenuated.

## TABLE 2

| Mutant | Sequence (470-484) | Viability |
|---|---|---|
| (Leon | AUCCUAACCAUGGAG | + ) |
| 1 | AauaUAACCAUGGAG | − |
| 2 | AaCaUAACCAUGGAG | − |
| 3 | AcuCUAAaaAUGagG | − |
| 7 | AUCCUAACCAUGaAG | + |
| 8 | AUuCUAACCAUGaAG | + |
| 9 | AUgCUAACCAUGcAG | − |
| 11 | AUuCcUAACCAUgGGAG | − |
| 12 | AUAG | − |
| 13 | AUCCUAACCcUGGAG | + |
| 14 | AUCCUAACCAcGGAG | + |

Mutant 7 (Table 2) was constructed by hybridising oligonucleotide 7 in Table 1 to the single stranded cloned DNA fragment in M13 phage. This oligonucleotide is complementary to the region 475-492 except at the position to be mutated, where the base complementary to the desired mutation is incorporated. In other words, oligonucleotide 7 contains U instead of C at the position complementary to base 482. The hybridised M13 and oligonucleotide DNA were incubated in a reaction mixture containing DNA precursors and the enzymes DNA polymerase and DNA ligase. After incubation for one hour at 37°C, closed circular DNA was isolated from this mixture by agarose gel electrophosesis. This DNA was then used to transform E coli mutS or mutL (deficient in DNA mismatch repair) which were then plated out on a lawn of E coli JM101.

M13 plaques which arose on this lawn of E coli were picked and propagated and single stranded M13 phage DNA isolated. The DNAs were then sequenced using the method of Sanger and those with the desired mutation were identified. From these, batches of replicative form double stranded DNA were prepared and the HindIII-SstI fragment containing 751 base pairs of infectious poliovirus cDNA, which incorporates the mutation, was recovered.

The mutated cDNA fragment was then reintroduced into a derivative of pVN23 which had been modified to include a T7 promoter. Live virus was recovered from the mutated full length cDNA by the production of a positive sense RNA transcript from the T7 promoter in vitro (Van der Werf et al) which was applied to Hela cells in tissue culture using standard techniques (Koch). After 4 to 6 days incubation a cytopathic effect was observed and virus could be recovered from the supernatant.

Recovered virus was plaque purified and propagated in Hela cells. This virus pool was used for the preparation of RNA on which the sequence of the virus mutant was verified using primer extension nucleotide sequencing. A portion of the pool was also used to assay neurovirulence using techniques described previously. The LD50 for mutant 7 was $7.5 \times 10^6$ pfu (compared to $>2 \times 10^7$ pfu for the vaccine derivative and $<6 \times 10^2$ pfu for the neurovirulent progenitor derivative), indicating that this mutation has a definite attenuating effect on the virus.

This procedure was repeated for oligonucleotides 1 to 3, 9 and 11 to 14. Testing of virus recovered from mutant cDNA 13 (i.e. A to C at 479) revealed that this mutation also had a definite attenuating effect on virulence (LD50 = $9.1 \times 10^6$). Conversely, testing of virus recovered from mutant cDNA 14 revealed that this mutation (i.e. U to C at 480) had little or no effect on neurovirulence (LD50 = $<7 \times 10^4$). Attempts to recover virus from mutant cDNAs 1, 2, 3, 9, 11 and 12 resulted in failure, indicating that they contain mutation(s) lethal for the virus.

The procedure was also repeated for oligonucleotide 8. Mutant cDNA 8 was constructed which contained both the mutations which are separately attenuating in the vaccine strain (i.e. C to U at 472) and in mutant 7 (i.e. G to A at 482). Virus recovered from this double mutant cDNA was tested for neurovirulence and found to have an LD50 of $>4.8 \times 10^7$ pfu. This double mutant is therefore more attenuated than either the vaccine strain or mutant 7.

**Claims**
**Claims for the following Contracting States : AT, BE, CH, LI, DE, FR, IT, LU, NL, SE**

1. An attenuated enterovirus or rhinovirus having an attenuating mutation at least at a position which is, or corresponds with, position 479 and/or 482 of the genome of poliovirus type 3 Leon strain.

2. An attenuated virus according to claim 1, which is a poliovirus.

3. An attenuated virus according to claim 2, which is a type 1 poliovirus.

4. An attenuated virus according to claim 2, which is a type 2 poliovirus.

5. An attenuated virus according to claim 2, which is a type 3 poliovirus.

6. An attenuated virus according to any one of claims 2 to 5 in which the base at position 479 or at a said corresponding position is cytosine.

7. An attenuated virus according to any one of claims 2 to 6 in which the base at position 482 or at a said corresponding position is adenine.

8. An attenuated virus according to claim 1, which also has an attenuating mutation at a position which is, or which corresponds with, position 472 of the genome of poliovirus type 3 Leon strain.

9. An attenuated virus according to claim 8, which is a poliovirus which has the base U at position 472 or at a said corresponding position.

**10.** A process for the preparation of an attenuated enterovirus or rhinovirus as defined in claim 1, which process comprises:

(i) introducing the or each desired mutation by site-directed mutagenesis into a sub-cloned region, which includes the or each position it is wished to mutate, of a cDNA copy of the genome of an enterovirus or rhinovirus;

(ii) reintroducing the thus modified region into the complete cDNA from which the region was derived; and

(iii) obtaining live virus from the cDNA thus obtained.

**11.** A pharmaceutical composition comprising a pharmaceutically acceptable carrier or diluent and an attenuated virus as claimed in claim 1.

**12.** An attenuated virus as claimed in claim 1 for use as a vaccine.

**Claims for the following Contracting States : ES, GR**

**1.** A process for the preparation of an attenuated enterovirus or rhinovirus having an attenuating mutation at least at a position which is, or corresponds with, position 479 and/or 482 of the genome of poliovirus type 3 Leon strain, which process comprises:

(i) introducing the or each desired mutation by site-directed mutagenesis into a sub-cloned region, which includes the or each position it is wished to mutate, of a cDNA copy of the genome of an enterovirus or rhinovirus;

(ii) reintroducing the thus modified region into the complete cDNA from which the region was derived; and

(iii) obtaining live virus from the cDNA thus obtained.

**2.** A process according to claim 1, in which the virus is a poliovirus.

**3.** A process according to claim 2, in which the virus is a type 1 poliovirus.

**4.** A process according to claim 2, in which the virus is a type 2 poliovirus.

**5.** A process according to claim 2, in which the virus is a type 3 poliovirus.

**6.** A process according to any one of claims 2 to 5 in which in the virus the base at position 479 or at a said corresponding position is cytosine.

**7.** A process according to any one of claims 2 to 6 in which in the virus the base at position 482 or at a said corresponding position is adenine.

**8.** A process according to claim 1, in which the virus also has an attenuating mutation at a position which is, or which corresponds with, position 472 of the genome of poliovirus type 3 Leon strain.

**9.** A process according to claim 8, in which the virus is a poliovirus which has the base U at position 472 or at a said corresponding position.

**10.** A process for preparing a pharmaceutical composition comprising admixing a pharmaceutically acceptable carrier or diluent and an attenuated virus prepared by a process as claimed in claim 1.

**Patentansprüche**
**Patentansprüche für folgende Vertragsstaaten : AT, BE, CH, DE, FR, IT, LI, LU, NL, SE**

**1.** Attenuierter Enterovirus oder Rhinovirus, der an mindestens einer Position, die die Position 479 und/oder 482 des Genoms vom Poliovirus Typ 3 Leonstamm ist oder dieser entspricht, eine attenuierende Mutation aufweist.

**2.** Attenuierter Virus nach Anspruch 1, dadurch gekennzeichnet, daß er ein Poliovirus ist.

**3.** Attenuierter Virus nach Anspruch 2, dadurch gekennzeichnet, daß er ein Typ 1 Poliovirus ist.

**4.** Attenuierter Virus nach Anspruch 2, dadurch gekennzeichnet, daß er ein Typ 2 Poliovirus ist.

**5.** Attenuierter Virus nach Anspruch 2, dadurch gekennzeichnet, daß er ein Typ 3 Poliovirus ist.

**6.** Attenuierter Virus nach einem der Ansprüche 2 bis 5, dadurch gekennzeichnet, daß die Base an Position 479 oder an einer entsprechenden Position Cytosin ist.

**7.** Attenuierter Virus nach einem der Ansprüche 2 bis 6, dadurch gekennzeichnet, daß die Base an Position 482 oder an einer entsprechenden Position Adenin ist.

**8.** Attenuierter Virus nach Anspruch 1, dadurch gekennzeichnet, daß er auch an einer Position, die die Position 472 des Genoms vom Poliovirus Typ 3 Leonstamm ist oder dieser entspricht, eine attenuierende Mutation aufweist.

**9.** Attenuierter Virus nach Anspruch 8, dadurch gekennzeichnet, daß er ein Poliovirus ist, der die Base U an Position 472 oder an einer entsprechenden Position besitzt.

**10.** Verfahren zur Herstellung eines attenuierten Enterovirus oder Rhinovirus nach Anspruch 1, dadurch gekennzeichnet, daß es umfaßt:
(i) Einführen der oder jeder gewünschten Mutation durch Sitedirected-Mutagenese einer cDNA-Kopie des Genoms eines Enterovirus oder Rhinovirus in eine subklonierte Region, die die oder jede Position, von der gewünscht wird, daß sie mutiert, enthält;
(ii) Wiedereinführen der so modifizierten Region in die komplette cDNA, aus der die Region stammt; und
(iii) Gewinnen des lebenden Virus aus der so erhaltenen cDNA.

**11.** Pharmazeutische Zusammensetzung, dadurch gekennzeichnet, daß sie einen pharmazeutisch annehmbaren Träger oder Verdünner und einen attenuierten Virus gemäß Anspruch 1 umfaßt.

**12.** Attenuierter Virus gemäß Anspruch 1 zur Verwendung als Impfstoff.

**Patentansprüche für folgende Vertragsstaaten : ES, GR**

**1.** Verfahren zur Herstellung eines attenuierten Enterovirus oder Rhinovirus, der an mindestens einer Position, die die Position 479 und/oder 482 des Genoms vom Poliovirus Typ 3 Leonstamm ist oder dieser entspricht, eine attenuierende Mutation aufweist, dadurch gekennzeichnet, daß es umfaßt:
(i) Einführen der oder jeder gewünschten Mutation durch Sitedirected-Mutagenese einer cDNA-Kopie des Genoms eines Enterovirus oder Rhinovirus in eine subklonierte Region, die die oder jede Position, von der gewünscht wird, daß sie mutiert, enthält;
(ii) Wiedereinführen der so modifizierten Region in die komplette cDNA, aus der die Region stammt; und
(iii) Gewinnen des lebenden Virus aus der so erhaltenen cDNA.

**2.** Verfahren nach Anspruch 1, dadurch gekennzeichnet, daß der Virus ein Poliovirus ist.

**3.** Verfahren nach Anspruch 2, dadurch gekennzeichnet, daß der Poliovirus ein Typ 1 Poliovirus ist.

**4.** Verfahren nach Anspruch 2, dadurch gekennzeichnet, daß der Virus ein Typ 2 Poliovirus ist.

**5.** Verfahren nach Anspruch 2, dadurch gekennzeichnet, daß der Virus ein Typ 3 Poliovirus ist.

**6.** Verfahren nach einem der Ansprüche 2 bis 5, dadurch gekennzeichnet, daß in den Virus die Base an Position 479 oder an einer entsprechenden Position Cytosin ist.

**7.** Verfahren nach einem der Ansprüche 2 bis 6, dadurch gekennzeichnet, daß in dem Virus die Base an Position 482 oder an einer entsprechenden Position Adenin ist.

8

**8.** Verfahren nach Anspruch 1, dadurch gekennzeichnet, daß der Virus auch eine attenuierende Mutation an einer Position aufweist, die die Position 472 des Genoms vom Poliovirus Typ 3 Leonstamm ist oder ihr entspricht.

**9.** Verfahren nach Anspruch 8, dadurch gekennzeichnet, daß der Virus ein Poliovirus ist, der die Base U an Position 472 oder einer entsprechenden Position aufweist.

**10.** Verfahren zur Herstellung einer pharmazeutischen Zusammensetzung, dadurch gekennzeichnet, daß man einen pharmazeutisch annehmbaren Träger oder Verdünner und einen nach einem Verfahren gemäß Anspruch 1 hergestellten Virus mischt.

**Revendications**
**Revendications pour les Etats contractants suivants : AT, BE, CH, DE, FR, IT, LI, LU, NL, SE**

**1.** Entérovirus ou rhinovirus atténué, dans lequel on a introduit une mutation, produisant un virus atténué, au niveau d'au moins une position qui est, ou qui correspond à, la position 479 et/ou 482 du génome du poliovirus type 3 souche Leon.

**2.** Virus atténué selon la revendication 1, qui est un poliovirus.

**3.** Virus atténué selon la revendication 2, qui est un poliovirus type 1.

**4.** Virus atténué selon la revendication 2, qui est un poliovirus type 2.

**5.** Virus atténué selon la revendication 2, qui est un poliovirus type 3.

**6.** Virus atténué selon l'une quelconque des revendications 2 à 5, dans lequel la base à la position 479 ou à ladite position correspondante est la cytosine.

**7.** Virus atténué selon l'une quelconque des revendications 2 à 6, dans lequel la base à la position 482 ou à ladite position correspondante est l'adénine.

**8.** Virus atténué selon la revendication 1, ayant subi également une mutation produisant un virus atténué à la position qui est, ou qui correspond à la, position 472 du génome du poliovirus type 3, souche Leon.

**9.** Virus atténué selon la revendication 8, qui est un poliovirus comprenant la base U à la position 472 ou à ladite position correspondante.

**10.** Procédé de préparation d'un entérovirus ou rhinovirus atténué, selon la revendication 1, ledit procédé comprenant les étapes consistant à :
(i) introduire la ou chaque mutation voulue par mutagenèse dirigée dans une région sous-clonée, qui comprend la ou chaque position que l'on veut soumettre à une mutation, d'une copie d'ADNc du génome d'un entérovirus ou rhinovirus;
(ii) réintroduire la région ainsi modifiée dans l'ADNc complet dont provient la région ; et
(iii) obtenir le virus vivant à partir de l'ADNc ainsi obtenu.

**11.** Composition pharmaceutique comprenant un véhicule ou diluant pharmaceutiquement acceptable et un virus atténué selon la revendication 1.

**12.** Virus atténué selon la revendication 1, utilisable comme vaccin.

**Revendications pour les Etats contractants suivants : ES, GR**

**1.** Procédé de préparation d'un entérovirus ou rhinovirus atténué, dans lequel on a introduit une mutation, produisant un virus atténué, au niveau d'au moins une position qui est, ou qui correspond à, la position 479 et/ou 482 du génome du poliovirus type 3 souche Leon, ledit procédé comprenant les étapes consistant à :

(i) introduire la ou chaque mutation voulue par mutagenèse dirigée dans une région sous-clonée, qui comprend la ou chaque position que l'on veut soumettre à une mutation, d'une copie d'ADNc du génome d'un entérovirus ou rhinovirus ;

(ii) réintroduire la région ainsi modifiée dans l'ADNc complet dont provient la région ; et

(iii) obtenir le virus vivant à partir de l'ADNc ainsi obtenu.

2. Procédé selon la revendication 1, dans lequel le virus est un poliovirus.

3. Procédé selon la revendication 2, dans lequel le virus est un poliovirus type 1.

4. Procédé selon la revendication 2, dans lequel le virus est un poliovirus type 2.

5. Procédé selon la revendication 2, dans lequel le virus est un poliovirus type 3.

6. Procédé selon l'une quelconque des revendications 2 à 5, dans lequel, dans le virus, la base à la position 479 ou à ladite position correspondante est la cytosine.

7. Procédé selon l'une quelconque des revendications 2 à 6, dans lequel, dans le virus, la base à la position 482 ou à ladite position correspondante est l'adénine.

8. Procédé selon la revendication 1, dans lequel le virus a subi également une mutation produisant un virus atténué à la position qui est, ou qui correspond à la, position 472 du génome du poliovirus type 3, souche Leon.

9. Procédé selon la revendication 8, dans lequel le virus est un poliovirus comprenant la base U à la position 472 ou à ladite position correspondante.

10. Procédé de préparation d'une composition pharmaceutique, consistant à mélanger un véhicule ou diluant pharmaceutiquement acceptable et un virus atténué préparé par un procédé selon la revendication 1.